# EUROPEAN PATENT SPECIFICATION

(11) **EP 2 014 323 B1**
(45) Date of publication and mention of the grant of the patent: **06.10.2010**
(21) Application number: 08010951.5
(22) Date of filing: 17.06.2008
(51) Int. Cl.: A61M 5/20, A61C 5/06

(54) **Cartridge liquid injector**
Kartuschenflüssigkeitsinjektor
Injecteur de liquide de cartouche

(30) Priority: 10.07.2007 JP 2007180933
(43) Date of publication of application: 14.01.2009
(73) Proprietor: NIPRO CORPORATION, Osaka-shi, Osaka 531-8510 (JP)
(72) Inventor: Takahashi, Nobuhiro, Hanamaki-shi Iwate 028-3111 (JP); Takahashi, Hidenori, Osaka-shi, Osaka 531-8510 (JP); Aihara, Minoru, Osaka-shi, Osaka 531-8510 (JP)
(74) Representative: Prüfer & Partner GbR European Patent Attorneys

(56) References cited:
- EP-A- 1 462 134
- WO-A-96/26754
- WO-A-03/080160
- WO-A-2004/108193
- US-A- 5 807 334
- US-A1- 2006 178 640

## Description

### Field of the Invention

The present invention relates to a cartridge liquid injector.

### Description of the Background Art

Generally, a cartridge liquid injector is heavily used as a liquid injector for an injection, with a needle being attached to a tip end opening of a cartridge.

A conventional electrically-driven injection device of such a type includes a pinion turned by a motor with reduction device through a power transmission gear mechanism and a plunger having a rack engaged with the pinion. For example, in injecting an injection into oral cavity, the plunger is moved forward at an extremely low speed. In addition, after an injection operation is completed, rotation of the motor with reduction device is reversed so as to move backward the plunger.

Returning the plunger by reversing the rotation of the motor with reduction device, however, takes too long a time. Therefore, the plunger and a drive mechanism for the same are disconnected from each other so that the plunger can freely be pushed back with hands (see, for example, Japanese Patent Laying-Open No. 2001-070444).

The conventional device, however, gives rise to a problem of insanitation because the plunger is returned with hands, and development of a cartridge liquid injector in which the plunger can quickly be returned by being electrically driven has been demanded.

US 5,807,334 A shows a fluid dispenser which includes a hollow housing having an elongate chamber formed therein. A power supply is located within the chamber of the housing, the power suppl being in electrical communication with a motor also located within the chamber of the housing. A switch, accessable from outside the housing, is in electrical communication with the power supply and motor for selectively operating the motor from a non-operable condition to an operable condition. A gear train is driven by a drive shaft of the motor, the gear train being located within the chamber of the housing and having a plurality of gears. At least one of the gears defines a slow speed gear which is rotatable at a relative slow rate of speed and at least another of the gears defines a fast speed gear which is rotatable at a relatively fast rate of speed. A rack member is selectively engageable with one of the slow and fast speed gears for moving the rack member linearly from a position in which the end portion is spaced from a carpule containing fluid to be dispensed to a position in which the end portion engages a carpule piston so as to effect the dispensing of fluid from the carpule at a controlled rate.

### SUMMARY OF THE INVENTION

The present invention was made in view of the above, and an object of the present invention is to provide a cartridge liquid injector in which a plunger can quickly be returned by being electrically driven.

In order to achieve the object above, a cartridge liquid injector according to claim 1 is provided.

With such an arrangement, according to the present invention, the reduction ratio switching portion can switch the reduction ratio of the reduction portion such that the reduction ratio in pulling back the plunger is lower than the reduction ratio in pushing forward the same. Thus, a speed of pushing forward the plunger can be low and a speed of pulling back the plunger can be high.

In addition, in the cartridge liquid injector according to the present invention, the reduction ratio switching portion is formed to switch the reduction ratio by switching an engaged state of gears in a reduction gear group forming the reduction portion.

With such an arrangement, according to the present invention, the reduction ratio can appropriately and readily be switched by switching an engaged state of the gears in the reduction gear group.

In addition, in the cartridge liquid injector according to the present invention, the reduction ratio switching portion is formed to switch the reduction ratio in response to attachment and removal of the cartridge to and from an injector main body.

With such an arrangement, according to the present invention, the speed of the plunger can automatically be set to an appropriate speed as a result of switching of the reduction ratio by the reduction ratio switching portion in response to attachment and removal of the cartridge to and from the injector main body.

In addition, in the cartridge liquid injector according to the present invention, the drive motor is formed such that rotation in forward and reverse directions thereof is switched in response to attachment and removal of the cartridge to and from an injector main body.

With such an arrangement, according to the present invention, the plunger can appropriately be pushed forward or pulled back as a result of switching between rotations in forward and reverse directions of the drive motor in response to attachment and removal of the cartridge to and from the injector main body.

Thus, according to the cartridge liquid injector of the present invention, the plunger can quickly be pulled back by being electrically driven.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a cross-sectional view showing an embodiment of a cartridge liquid injector of the present invention.
Fig. 2 is a cross-sectional view similar to Fig. 1, showing a state where a cartridge is attached.
Fig. 3 is an enlarged perspective view of a reduction ratio switching portion of the cartridge liquid injector of the present invention.
Fig. 4 is an enlarged perspective view similar to Fig. 3, of the reduction ratio switching portion of the cartridge liquid injector of the present invention.
Fig. 5 is a block diagram of a portion controlling a drive motor of the cartridge liquid injector of the present invention.

### DESCRIPTION OF THE PREFERRED EMBODIMENTS

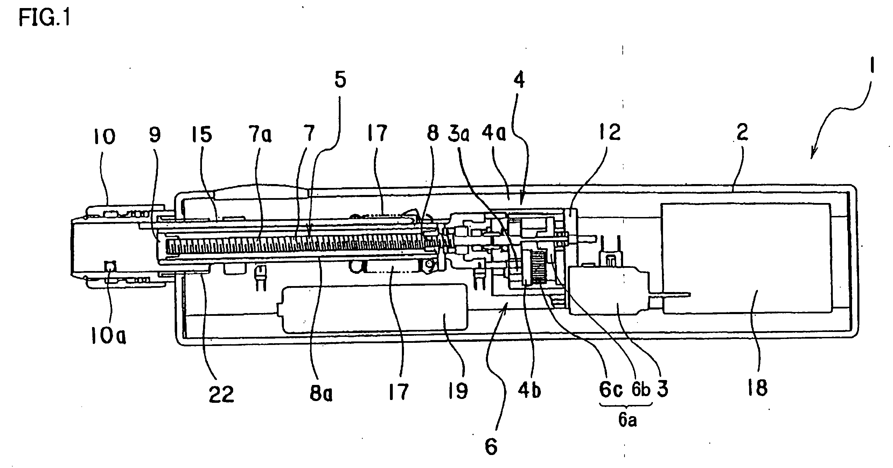

An embodiment of the present invention will be described hereinafter with reference to Figs. 1 to 5.

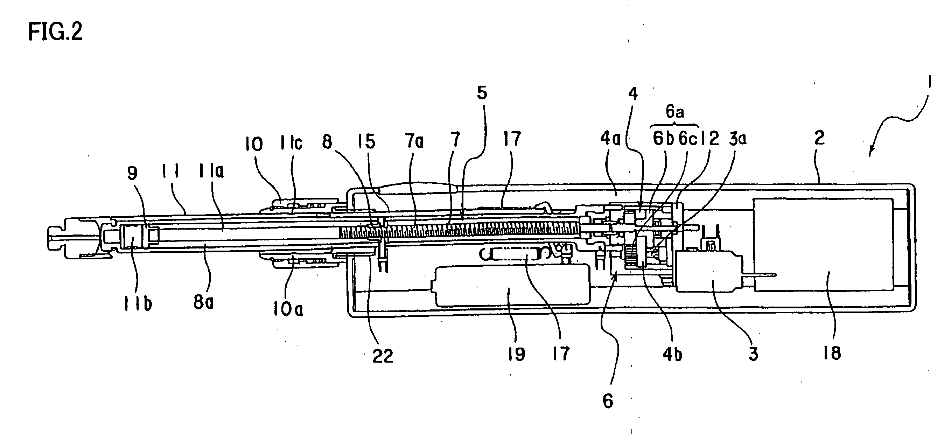

As shown in Figs. 1 and 2, cartridge liquid injector 1 according to the present embodiment is formed such that an apparatus main body 2 like a long case contains each main component.

In apparatus main body 2, a drive motor 3 implemented by a DC motor or the like rotatable in forward and reverse directions, for applying driving force, is fixed on a base end side. On an output side of drive motor 3, a reduction portion 4 reducing a speed of rotational output of drive motor 3 and a linear motion conversion portion 5 converting rotational output of reduction portion 4 to linear motion are successively coupled.

Reduction portion 4 is implemented in a gear box 12 by a gear group 4a in which a plurality of gears are coupled. A part of gear group 4a serves as a moving gear group 6a movable in an axial direction, that implements a part of a reduction ratio switching portion 6 which will be described later.

An output shaft of gear group 4a serves as a long rotation shaft 7 implementing linear motion conversion portion 5. Rotation shaft 7 is formed to have a length from reduction portion 4 to a tip end of apparatus main body 2. Linear motion conversion portion 5 is formed of a thread portion 7a formed on an outer circumferential surface of rotation shaft 7, a nut 8 screwed thereto, and a sleeve 8a. Though nut 8 is incapable of turning, it is attached movably in the axial direction in such a manner that a direction of movement is switched depending on rotation of rotation shaft 7 in forward and reverse directions.

Pipe-like sleeve 8a in which rotation shaft 7 is loosely inserted is fastened to nut 8 on the outer side in apparatus main body 2. A plunger 9 is fastened to a tip end of sleeve 8a with such a method as screwing.

A cylindrical support element 22 in a cylindrical shape is fastened to a tip end wall portion of apparatus main body 2 in a manner penetrating the tip end wall portion. Cylindrical support element 22 supports an outer circumferential portion of plunger 9 and sleeve 8a in a manner movable in the axial direction. A cylindrical cartridge holder attachment and removal mechanism 10 is fixed to an outer circumferential portion of cylindrical support element 22 concentrically with plunger 9 with such a method as screwing.

As shown in Fig. 1, in a most pulled-back state, a tip end of plunger 9 faces in cartridge holder attachment and removal mechanism 10. As shown in Fig. 2, a cartridge holder 11 in a long cylindrical shape is attached inside cartridge holder attachment and removal mechanism 10.

A cartridge 11a in a long cylindrical shape is inserted in cartridge holder 11. A liquid is hermetically stored in cartridge 11a and an injection plug 11b is provided at a base end portion in a manner movable in the axial direction. Cartridge holder 11 is inserted in cartridge holder attachment and removal mechanism 10 until injection plug 11b of cartridge 11a abuts plunger 9. Cartridge holder 11 is attached to cartridge holder attachment and removal mechanism 10 by engaging a click 10a provided on an inner circumferential surface of cartridge holder attachment and removal mechanism 10 with a recess portion 11c formed on an outer circumferential surface of cartridge holder 11.

When rotation shaft 7 is turned in a forward direction while cartridge 11 a and cartridge holder 11 are attached to cartridge holder attachment and removal mechanism 10, plunger 9 is pushed forward by linear motion conversion portion 5. Thus, injection plug 11b of cartridge 11a is moved forward. When rotation shaft 7 is turned in a reverse direction, plunger 9 is pulled back by linear motion conversion portion 5.

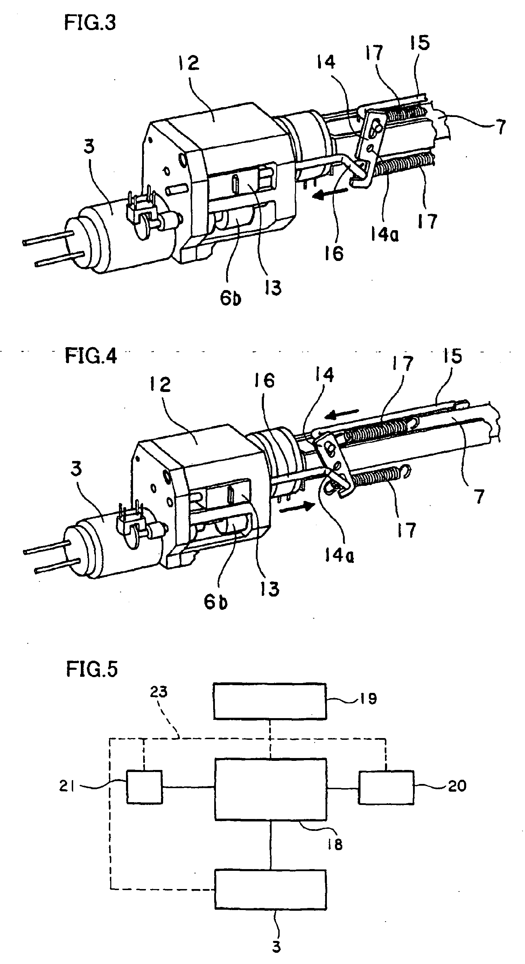

Reduction ratio switching portion 6 serves to switch the reduction ratio such that the reduction ratio of reduction portion 4 in pulling back plunger 9 is lower than that in pushing forward the same. As shown in Figs. 3 and 4, reduction ratio switching portion 6 is implemented by moving gear group 6a movable in the axial direction by means of moving element 13, that is provided in gear box 12. In the present embodiment shown in Figs. 1 to 4, moving gear group 6a is formed of two gears 6b and 6c.

As shown in Figs. 2 and 4, the state where moving element 13 has moved to the tip end side of rotation shaft 7 is the pushed forward state of plunger 9. As gear 6b attached to rotation shaft 7 is engaged with gear 4b attached to a drive shaft 3 a located on the drive source side relative to rotation shaft 7, driving force is transmitted.

As shown in Figs. 1 and 3, the state where moving element 13 has moved to the base end side of rotation shaft 7 is the pulled back state of plunger 9. As a result of movement of moving gear group 6a, gear 6b attached rotation shaft 7 is engaged with gear 6c attached to drive shaft 3 a located on the drive source side relative to rotation shaft 7 and driving force is transmitted.

In the present embodiment, the reduction ratio of reduction portion 4 determined by a coupling state of gear groups 4a and 6a, that is, coupling of two gears 6b and 6c, in pulling back is set lower than the reduction ratio of reduction portion 4 determined by a coupling state of gear groups 4a and 6a, that is, coupling of two gears 4b and 6b, in pushing forward.

Reduction ratio switching portion 6 may be formed such that the reduction ratio is automatically switched in response to attachment and removal of cartridge 11a and cartridge holder 11 to and from cartridge holder attachment and removal mechanism 10 fixed to apparatus main body 2. Specifically, in a position in the rear of gear box 12 shown in Figs. 1 and 2, a swing link 14 swinging around a swing center 14a is provided on the tip end side of gear box 12. A cartridge detection bar 15 having a length from one end of swing link 14 to the inside of cartridge holder attachment and removal mechanism 10 is provided. A coupling bar 16 couples the other end of swing link 14 and moving element 13 within gear box 12 to each other. As shown in Fig. 3, springs 17 applying elastic force to move swing link 14 in such a direction that moving element 13 is moved to a position in a state where plunger 9 is pulled back are connected to opposing ends of swing link 14.

Moving gear group 6a, that is, two gears 6b and 6c, of reduction ratio switching portion 6 is attached to moving element 13. Therefore, when cartridge 11a and cartridge holder 11 are not attached, as shown in Figs. 3 and 1, elastic force of spring 17 acts to move swing link 14 and the reduction ratio of reduction portion 4 is set to the reduction ratio in pulling back plunger 9. When cartridge 11a and cartridge holder 11 are attached to cartridge holder attachment and removal mechanism 10, as shown in Figs. 4 and 2, cartridge detection bar 15 is pushed into apparatus main body 2 against the elastic force of spring 17. Accordingly, swing link 14 and coupling bar 16 are moved and the reduction ratio of reduction portion 4 is set to the reduction ratio in pushing forward plunger 9.

In addition, in the present embodiment, drive motor 3 may be formed such that rotation in forward and reverse directions thereof may be switched in response to attachment and removal of cartridge 1 1 a and cartridge holder 11 to and from cartridge holder attachment and removal mechanism 10. Specifically, as shown in Fig. 5 showing an exemplary block configuration of a control circuit as well as Figs. 1 and 2, apparatus main body 2 contains a control unit 18 formed with a CPU, a bus and the like, a battery 19, and an attachment and removal sensor 20 detecting a moved state, for example, of moving element 13 and swing link 14 and detecting whether cartridge 11a and cartridge holder 11 are attached to cartridge holder attachment and removal mechanism 10 or not. As attachment and removal sensor 20 detects attachment and removal of cartridge 11a and cartridge holder 11, control unit 18 issues an instruction to cause drive motor 3 to rotate in forward and reverse directions.

Moreover, cartridge liquid injector 1 itself may be turned on and off by using a switch 21. Battery 19 supplies electric power to each portion requiring power supply through an electric line 23.

An operation of the cartridge liquid injector of the present embodiment will now be described.

In a state that cartridge 11a and cartridge holder 11 are not attached to cartridge holder attachment and removal mechanism 10 as shown in Figs. 1 and 3, cartridge 11a and cartridge holder 11 are pushed into cartridge holder attachment and removal mechanism 10 and attached thereto as shown in Figs. 2 and 4. Here, cartridge detection bar 15 is pushed into apparatus main body 2 against the elastic force of spring 17. Accordingly, swing link 14 and coupling bar 16 are moved. Moving element 13 is also moved to the tip end side together with moving gear group 6a, and gear 6b attached to rotation shaft 7 is engaged with gear 4b attached to drive shaft 3 a located on the drive source side relative to rotation shaft 7. Thus, the reduction ratio of reduction portion 4 is set to the reduction ratio in pushing forward plunger 9.

When switch 21 is switched on, battery 19 supplies electric power to each portion. Attachment and removal sensor 20 detects attachment of cartridge 11a and cartridge holder 11 to cartridge holder attachment and removal mechanism 10 and outputs such a fact to control unit 18. Control unit 18 issues an instruction to cause drive motor 3 to rotate in the forward direction.

Thus, drive motor 3 rotates in the forward direction. Rotation shaft 7 turns at low speed at the reduction ratio in pushing forward plunger 9, that is set in advance by reduction portion 4. Linear motion conversion portion 5 pushes forward plunger 9. As cartridge 11 a and cartridge holder 11 are immovably held by apparatus main body 2 as a result of engagement between click 10a and recess portion 11c with each other, plunger 9 is pushed forward and injection plug 11b moves forward. For example, when a needle (not shown) is attached to the tip end of cartridge 11a, injection plug 11b moves forward to make an injection of a liquid within cartridge 11a. In a state that plunger 9 is pushed forward most, idling of drive motor 3 implemented by a DC motor may be detected, so that rotation of drive motor 3 is stopped.

Thereafter, cartridge 11a and cartridge holder 11 are removed from cartridge holder attachment and removal mechanism 10. Then, as shown in Fig. 3, cartridge detection bar 15 is set to a free state. Swing link 14 is forcibly pivoted by the elastic force of spring 17, and cartridge detection bar 15 returns to the tip end side. Moving element 13 coupled to coupling bar 16 is moved toward the base end side together with moving gear group 6a. Gear 6b attached to rotation shaft 7 is engaged with gear 6c attached to drive shaft 3a located on the drive source side relative to rotation shaft 7. Thus, the reduction ratio of reduction portion 4 is set to the reduction ratio in pulling back plunger 9.

In addition, attachment and removal sensor 20 detects removal of cartridge 11a and cartridge holder 11 from cartridge holder attachment and removal mechanism 10 and outputs a signal to control unit 18. Control unit 18 issues an instruction to cause drive motor 3 to rotate in the reverse direction. Thus, drive motor 3 rotates in the reverse direction. Rotation shaft 7 turns at high speed at the reduction ratio in pulling back plunger 9, that is set in advance by reduction portion 4. Linear motion conversion portion 5 automatically pulls back plunger 9 at high speed.

In a state that plunger 9 is pulled back most, idling of drive motor 3 implemented by a DC motor may be detected, so that rotation of drive motor 3 is stopped. As plunger 9 is pulled back, cartridge liquid injector 1 returns to a state shown in Fig. 1. Therefore, cartridge holder 11 in which new cartridge 11a is inserted is again attached to cartridge holder attachment and removal mechanism 10, and an operation to move injection plug 11b forward can be repeated.

It is noted that the present invention is not limited to the embodiment above and may be modified as necessary.

For example, an electromagnetic actuator is directly connected to moving element 13, instead of swing link 14, cartridge detection bar 15, coupling bar 16, and spring 17. The electromagnetic actuator may be operated to move moving element 13, depending on an attachment and removal state of cartridge 11a and cartridge holder 11 to and from cartridge holder attachment and removal mechanism 10 detected by attachment and removal sensor 20.

In addition, any reduction portion and reduction ratio switching portion capable of reduction and switching of the reduction ratio by using combination of gears to be engaged may be employed, and therefore, various types of planetary gear mechanisms and gear shift mechanisms may be combined and applied as appropriate.

Although the present invention has been described and illustrated in detail, it is clearly understood that the same is by way of illustration and example only and is not to be taken by way of limitation, the scope of the present invention being interpreted by the terms of the appended claims.

## Claims

1. A cartridge liquid injector, comprising:
a drive motor (3) rotatable in forward and reverse directions, for applying driving force;
a reduction portion (4) reducing a speed of rotational output of said drive motor;
a linear motion conversion portion (5) converting rotational output of said reduction portion to linear motion;
a plunger (9) pushed forward or pulled back by said linear motion conversion portion (5), for moving forward an injection plug (11b) of a cartridge (11a); and
a reduction ratio switching portion (6) switching a reduction ratio of said reduction portion (4) such that the reduction ratio in pulling back said plunger (9) is lower than the reduction ratio in pushing forward said plunger (9), **characterized in that** said reduction ratio switching portion (6) switches the reduction ratio in response to attachment and removal of the cartridge (11a) to and from an injector main body.

2. The cartridge liquid injector according to claim 1, wherein
said reduction ratio switching portion (6) switches the reduction ratio by switching an engaged state of a gear in a reduction gear group forming the reduction portion (4).

3. The cartridge liquid injector according to any one of claims 1 to 2, wherein
said drive motor (3) is switched between rotations in forward and reverse directions in response to attachment and removal of the cartridge (11a) to and from an injector main body.

## Patentansprüche

1. Ein Kartuschen-Flüssigkeits-Injektor, der umfasst:
einen Antriebsmotor (3), der in Vorwärts- und Rückwärtsrichtungen rotierbar ist, zum Aufbringen von Antriebskraft;
ein Reduzierteil (4), das eine Geschwindigkeit der Rotationsausgabe des Antriebsmotors reduziert;
ein Linearbewegungs-Konvertierteil (5), das die Rotationsausgabe des Reduzierteils in eine Linearbewegung konvertiert;
einen Kolben (9), der durch das Linearbewegungs-Konvertierteil (5) vorgeschoben oder zurückgezogen wird, zum Vorwärtsbewegen eines Injektionspfropfens (11b) einer Kartusche (11a); und
ein Untersetzungsverhältnis-Schaltteil (6), das das Untersetzungsverhältnis des Reduzierteils (4) derart schaltet, dass das Untersetzungsverhältnis beim Zurückziehen des Kolbens (9) geringer ist als das Untersetzungsverhältnis beim Vorschieben des Kolbens (9), **dadurch gekennzeichnet, dass**
das Untersetzungsverhältnis-Schaltteil (6) das Untersetzungsverhältnis als Antwort auf das Anbringen und Entfernen der Kartusche (11a) an und von einem Injektor-Hauptkörper schaltet.

2. Der Kartuschen-Flüssigkeits-Injektor gemäß Anspruch 1, wobei
das Untersetzungsverhältnis-Schaltteil (6) das Untersetzungsverhältnis schaltet durch Schalten eines Eingriffszustands eines Zahnrads in einer Untersetzungsgetriebegruppe, die das Reduzierteil (4) bildet.

3. Der Kartuschen-Flüssigkeits-Injektor gemäß einem der Ansprüche 1 bis 2, wobei
der Antriebsmotor (3) zwischen Rotationen in Vorwärts- und Rückwärtsrichtungen geschaltet wird, als Antwort auf das Anbringen und das Entfernen der Kartusche (11a) an und von einem Injektor-Hauptkörper.

## Revendications

1. Injecteur de liquide de cartouche comprenant :
un moteur d'entraînement (3) pouvant tourner dans les directions avant et arrière, pour appliquer une force d'entraînement ;
une partie de réduction (4) réduisant une vitesse de sortie de rotation dudit moteur d'entraînement ;
une partie de conversion de mouvement linéaire (5) convertissant la sortie de rotation de ladite partie de réduction en mouvement linéaire ;
un piston plongeur (9) poussé en avant ou retiré par ladite partie de conversion de mouvement linéaire (5), pour déplacer vers l'avant une bougie d'injection (11b) d'une cartouche (11a) ; et
une partie de commutation de rapport de réduction (6) commutant un rapport de réduction de ladite partie de réduction (4) de sorte que le rapport de réduction en retirant ledit piston plongeur (9) est inférieur au rapport de réduction en poussant vers l'avant ledit piston plongeur (9), **caractérisé en ce que** :
ladite partie de commutation de rapport de réduction (6) commute le rapport de réduction en réponse à la fixation et au retrait de la cartouche (11a) à et d'un corps principal d'injecteur.

2. Injecteur de liquide de cartouche selon la revendication 1, dans lequel ladite partie de commutation de rapport de réduction (6) commute le rapport de réduction en commutant un état mis en prise d'un engrenage dans un groupe d'engrenages de réduction formant la partie de réduction (4).

3. Injecteur de liquide de cartouche selon l'une quelconque des revendications 1 à 2, dans lequel ledit moteur d'entraînement (3) est commuté entre des rotations dans les directions avant et arrière en réponse à la fixation et au retrait de la cartouche (11a) à et d'un corps principal d'injecteur.
